# EUROPEAN PATENT APPLICATION

(11) **EP 2 481 804 A1**
(43) Date of publication of application: **01.08.2012**
(21) Application number: 11000751.5
(22) Date of filing: 31.01.2011
(51) Int. Cl.: C12N 15/867

(54) **Lentiviral vector comprising at least two TAR elements**

(71) Applicant: Heinrich-Pette-Institut Leibniz-Institut für experimentelle Virologie-Stiftung bürgerlichen Rechts -, 20251 Hamburg (DE)
(72) Inventor: Hofmann-Sieber, Helga, Dr., 22455 Hamburg (DE); Hauber, Joachim, Prof., Dr., 22527 Hamburg (DE)
(74) Representative: UEXKÜLL & STOLBERG

(57) **Abstract**

The present application is directed to the field of vector technology, in particular, to vectors useful in gene therapy of retroviral diseases such as HIV. The present invention provides a vector comprising at least two TAR elements, e.g., a retroviral or lentiviral vector, which allows a highly specific expression of a nucleic acid operably linked to the TAR elements in cells comprising TAT, as well as methods of using the same, in particular, a reduced expression in the absence of TAT.

## Description

The present application is directed to the field of vector technology, in particular, to vectors useful in gene therapy of retroviral diseases such as HIV. The present invention provides a vector comprising at least two TAR elements, e.g., a retroviral or lentiviral vector, which allows a highly specific expression of a nucleic acid operably linked to the TAR elements in cells comprising TAT, as well as methods of using the same, in particular, a reduced expression in the absence of TAT..

### Background of the Invention

According to current numbers published by the Word Health Organization, there are more than 33 million people worldwide living with HIV, and approximately 2 million people are dying from AIDS-related illnesses every year. Although the virus has been known since 1985, there is still neither a cure for the disease nor an effective vaccine that can prevent infection. So far, medical treatment of HIV mainly concentrates on slowing down disease progression by inhibition of viral enzymes or virus cell fusion.

An alternative, gene therapeutic strategy to treat HIV which has been reported recently is based on a tailored site-specific recombinase (Tre-recombinase) (Sarkar et al., 2007). The recombinase is delivered to infected cells, where it targets a specific sequence in the HIV-long terminal repeat (LTR) region leading to excision of the integrated proviral DNA from the genome of the infected cell. This method does not only suppress viral replication, but it also opens up the possibility to eradicate HIV from an infected individual.

For gene therapeutic applications, the capacity to introduce a particular foreign or native gene into a cell and to control the expression of that gene is highly important. The introduction of a certain transgene into a host genome can be accomplished by a suitable nucleic acid vector. Viruses are highly suited to transfer genetic material into eukaryotic cells and to use the cellular machinery to express virally encoded genes. Hence, intensive research has focused on the potential of various viruses for gene delivery.

Over the past years, lentiviral vectors derived from HIV-1 have been developed that allow reliable gene transfer into a wide range of dividing and non-dividing cells, and that trigger long-term transgene expression. State-of-the-art lentiviral vectors hold high safety levels due to split packaging systems, self inactivating (SIN) vector design and introduction of upstream sequence elements (USE) (Dull et al., 1998; Schambach et al., 2007). These features minimize the risk of productive recombinants and insertional mutagenesis. The only gene expressed from these vectors is the delivered transgene, which is driven by an internal promoter. Lentiviral components of these vectors are reduced to a truncated LTR region (SIN-LTR), a splice donor and acceptor site, the packaging signal (ψ), the rev response element (RRE) and the central polypurine tract (PPT).

The wildtype LTR region of HIV-1 comprises the U3, R, and U5 region and occurs in two copies at the 5' and 3' end of the HIV DNA genome (5'-LTR/3'-LTR). It contains cis-acting promoter and enhancer elements and regulates viral gene transcription in a manner dependent on the viral Tat (transactivator of transcription) protein. Most of the enhancer elements as well as the core promoter can be found in the U3 region. Therefore, LTR promoter activity can be efficiently disabled by deletion of the majority of U3 (SIN-LTR). The R region comprises the trans-activating response (TAR) element and a PolyA signal. Both elements are essential for effective viral mRNA processing. The PolyA signal leads to termination of viral transcripts in the 3'-LTR while it is overread in the 5'-LTR.

The TAR element, on the other hand, acts on the level of transcription initiation and/or elongation by serving as the target sequence for Tat. After initiation of transcription, the TAR element leads to formation of an RNA stem loop structure at the 5' end of all viral transcripts, which halts viral mRNA transcription. Only in the presence of the HIV-1 trans-activating protein Tat, a complex with a cellular cofactors forms, and efficient mRNA transcription is effected by action of cellular RNA polymerase II (Karn, 1999).

In the elucidation of the function of the TAR element and the requirements for transactivation, early experiments by Selby et al., 1989, indicated that the TAR element needs to be located immediately downstream of the start of transcription. Later experiments by Churcher et al, 1995, which used templates carrying duplicated TAR elements with mutations inactivating the downstream element, however demonstrated that a TAR element can be functional placed up to 200 nt downstream of the start of transcription.

The TAR element has found use as a "decoy" element independent from its function in the context of a promotor. For this, the TAR element is produced as a short RNA fragment starting from a U6 promoter. This TAR RNA is localised in the nucleus of HIV infected cells and, there, competes with the HIV promotor for binding TAT. This leads to an inhibition of viral gene expression and thus, to inhibition of HIV propagation (Michienzi et al., 2002).

One major problem of gene therapeutic approaches is that constitutive expression of a heterologous transgene often leads to immunological or toxic reactions in the host. Therefore, targeting of transgene expression to the cells or tissue of interest is of great importance.

Several inducible promoters are known in the state of the art, which can, e.g., be dependent on tissue-specific or cell-specific factors or factors specific for a certain developmental stage. Amongst these are for example the glia cell-specific CD44 promoter, the albumin promoter that triggers long-term transgene expression in the liver and the rhodopsin promoter which induces high levels of transgene expression in photoreceptors (reviewed in Excors 2010).

For a gene therapeutic approach against HIV, the transgene can be put under control of a TAT-dependent promoter which is said to limit transgene expression to HIV infected cells (US 20060122139, Unwalla et al., 2004, Unwalla et al., 2008).

However, control of gene expression by TAR is known to be leaky (Park et al., 2009; Karn, 1999; Caruso, 1997). This is essential for HIV proliferation. It is believed that strong transcription of the provirus is dependent on a certain level of TAT protein accumulating in a cell after infection (Kam, 1999, Michienzi et al., 2002, Kao et al, 1987).

An alternative for HIV-dependent regulation of gene expression is the HIV Rev mechanism. This is based on the effect that viral transcripts that have been spliced a single time or that have not been spliced are not transferred from the nucleus into the cytoplasm. Only in the presence of the regulatory HIV protein Rev, these RNAs are also actively transported into the cytoplasm and processed further.

A transgene expression dependent on both TAT and Rev has been employed for inhibition of HIV by expression of cytotoxic or antiretroviral genes. This system was found to lead to a low basal level of expression of the transgene and to >300 fold induction in the presence of Tat and Rev (Ragheb et al., 1999).

### Description of the Invention

In light of the state of the art, the problem addressed by the present invention is the provision of an advantageous system which allows more selective, inducible expression of a nucleic acid, e.g., a transgene, in particular, an expression system which has a very low level of expression expression of the encoded nucleic acid in the absence of induction. This problem is solved by the subject matter of the claims.

In one aspect, the present invention provides a nucleic acid comprising at least two TAR elements operably linked to a nucleic acid sequence that is capable of providing a therapeutic effect in a cell infected with a retrovirus selected from a group comprising HIV.

It has surprisingly been found that disadvantages of the previously known inducible systems, such as the TAT-inducible system including a TAR element, can be avoided by operably linking two TAR elements to the nucleic acid. Compared to a single TAR promoter (e.g. HIV wildtype LTR), the duplicated TAR element, surprisingly, decreases basal promoter activity in the absence of the viral TAT transactivator. In the presence of TAT, promoter activity may increase or may be similar compared to a promotor comprising one TAR element.

The at least two TAR elements are suitable for selectively inducing the expression of the nucleic acid sequence operably linked to the TAR elements (e.g., a transgene), wherein expression of said nucleic acid sequence operably linked to the TAR elements is significantly decreased in a cell not comprising TAT compared to a nucleic acid comprising one TAR element operably linked to the nucleic acid sequence.

Previous results were contradictory on the question if TAT induced expression of a control gene operably linked to two TAR elements increased the expressed amount of control gene product. Additionally, while the previous literature tried to address this question with different experimental systems, the question of the effect of TAR duplication on basal expression in the absence of induction, i.e., in the absence of TAT, was never addressed. The prior art thus does not include any indications if the problem of providing a system as far as possible preventing expression of the gene of interest in the absence of an inducing stimulus could be solved by duplication of TAR.

If any conclusions can be drawn from the prior art, the result of Emiliani et al., 1996, that a virus carrying a TAR duplication is stable during replication, indicates that the control of expression is sufficiently leaky to allow replication.

Surprisingly, the present inventors could show that basal expression in the absence of TAT is significantly decreased with two TAR elements instead of one. Use of a two TAR elements instead of one can thus provide decreased expression of an operably linked nucleic acid in the absence of TAT. The experiments show that expression in the absence of TAT is decreased by a factor of at least 2, preferably, by a factor of at least 3, or by a factor of at least 4. In the presence of TAT, expression may be slightly reduced (at most by about a third) or even increased with two instead of one TAR elements. Expression levels may be quantified, e.g., as shown in the examples.

The at least two TAR elements are therefore suitable for inhibiting the expression of the nucleic acid sequence operably linked to the TAR elements in a cell not comprising TAT. The at least two TAR elements are also suitable for selectively inducing the expression of the nucleic acid sequence operably linked to the TAR elements, wherein said nucleic acid sequence operably linked to the TAR elements is expressed in a cell not comprising TAT with reduced levels or not at all. The invention also provides use of a nucleic acid comprising at least two TAR elements for decreasing the expression of a nucleic acid sequence operably linked to the TAR elements in a cell not comprising TAT. The invention provides an expression system allowing more selective, inducible expression of a nucleic acid, e.g., a transgene, wherein basal expression of the encoded nucleic acid in the absence of induction, e.g., in the absence of TAT, is significantly decreased.

In the context of the invention, "selective expression", "inhibition of expression" or "decreased basal expression" means that, preferably, there is a very low level of expression or no expression of the nucleic acid sequence, e.g., the transgene, in the absence of an inducer of expression such as TAT, i.e., the system is less leaky or not leaky. Reference for comparisons regarding decreased or reduced expression is a comparable or otherwise identical expression system wherein the nucleic acid sequence is operationally linked to one TAR element.

The nucleic acid sequence operably linked to the at least two TAR elements, e.g., the transgene, may be selectively expressed in a cell comprising TAT, such as an HIV-infected cell, or in a cell transduced with a vector providing expression of TAT. TAT may encoded by a different vector or the nucleic acid also comprising the TAR elements. Additional control elements may or may not be present, e.g. expression may additionally be controlled by Rev, an enhancer or an inducible promoter. In the context of the invention, HIV may be HIV-1. Consequently, TAT may be TAT derived from HIV-1.

In the context of the invention "a", "an" or "the" is meant to include the plural, e.g., an enhancer can be one or two or more enhancers. "At least two TAR elements" can mean two, three, four, five, six or more TAR elements. However, satisfactory results are obtained with two TAR elements, so the nucleic acid according to the invention preferably comprises two TAR elements operationally linked to a nucleic acid sequence. For example, in particular if the nucleic acid construct comprising the TAR elements is a lentiviral vector, the vector may comprise at least four TAR elements. The presence of one or more additional TAR elements in the SIN LTRs of a vector is however not considered essential.

In the context of the invention, the nucleic acid comprising at least two TAR elements may be RNA or DNA. The nucleic acid is recombinant, and may be present in isolated form. In particular, it may be a vector, such as an adenoviral vector, a retroviral vector, a lentiviral vector, or an alpha retroviral vector, e.g,. based on ASLV (avian sarcoma-leukosis virus). A vector typically includes further elements important for expression of a nucleic acid, e.g. a promoter, a TATA box, an enhancer, and/or a polyadenylation signal. The vector preferably is an inducible expression vector based on HIV, in particular, HIV-1. Vectors which may be modified to include two TAR elements are known in the art (e.g., Dull et al., 1998, Schambach et al. 2007).

The nucleic acid may also be a transcript obtained from transcription of the expression vector. As explained below, the TAR elements are preferably located in the 5' part of the transcript. The transcript may be a full length transcript of an operably linked nucleic acid comprising a poly-A sequence at the 3' end. Such a transcript may be obtained upon transcription in the presence of TAT.

Essential elements of a TAR element, e.g., minimal sequences, consensus sequences and functional mutations are well known in the state of the art, e.g., Karn, 1999, Michienzi et al., 2002, Roy et al., 1990. A wild type TAR element consists of 59 nucleotides. On the RNA

level, it forms an extremely stable RNAse resistant hairpin-structure. Important structural components for functionality of a TAR element are a hairpin-structure comprising a bulge structure in position +23 to +25, which is essential for binding of TAT. The consensus sequence of the bulge is UCU; possible different sequences without loss of function are UCN, UUN, UUU; wherein N is any nucleotide, i.e. only the U in position +23 is strictly required. An intact stem of the hairpin structure is also important. Mutations in the loop (position +31-+34; binding of cellular cofactors) and the stem (positions +9 to +12 and +49 to +52) reduce Tat-mediated transactivation (Roy et al., 1990). Thus, a functional TAR element preferably comprises the wildtype TAR sequence in these positions.

At least one TAR element employed in the invention may comprise a sequence encoding one hairpin RNA structure comprising a bulge structure capable of binding TAT. In a preferred embodiment, one TAR element does not comprise a second bulge structure capable of binding TAT. The bulge structure comprises at least one U residue, preferably, the sequences of the bulge structure described above. Wildtype TAR or a functional, optionally, mutated, TAR element capable of inhibiting expression of an operably linked sequence in the absence of TAT, and capable of mediating expression of said operably linked sequence in the presence of TAT may be employed in the invention. Non-functional sequences derived from TAR elements are not designated TAR-elements in the context of this invention. The sequence of the employed TAR elements may be identical or different. For example, at least one or two TAR elements may independently be a wildtype TAR element or a mutated functional TAR element.

At least one TAR element may comprise the consensus sequence 5'-GGGUCUCUCUGGUUAGACCAGAUCUGAGCCUGGGAGCUCUCUGGCUAACU-AGGGAACCC-3' (SEQ ID NO:1). One, to or more TAR elements may comprise a functional TAR element having at least 60% sequence identity, at least 70% sequence identity, at least 80% sequence identity, at least 90% sequence identity, or at least 95% sequence identity to SEQ ID NO: 1.

The TAR elements are operably linked to a nucleic acid sequence. The term "operably linked" includes reference to a functional linkage of at least two sequences. For example, this may include linkage between a promoter and a second sequence, wherein the promoter initiates and mediates transcription of the second sequence. Also included is linkage between a regulatory element such as TAR and a second nucleic acid expressed under the control of the regulatory element. Such sequences may additionally be operably linked to promoter sequences etc.

In one embodiment, the nucleic acid of the invention comprises a promoter operationally linked to at least two TAR elements operationally linked to the nucleic acid sequence (e.g., the transgene). Suitable promoters are known in the art. The nucleic acid comprising the at least two TAR elements is functionally linked to the promoter if the promoter enables transcription of at least a part of the nucleic acid sequence up to and including the TAR elements. The at least two TAR elements are thus located downstream of the start of transcription. The promoter may be derived from a retroviral LTR, e.g., an HIV or HIV-1 LTR, in particular, a promoter comprising the U3 sequence. A part of the R sequence of a retroviral LTR is linked to the promoter, which may provide a TAR element. The promoter may be a non-virus derived promoter, e.g., a constitutive promoter such as the hsp70 promoter, or a cell-specific promoter. A promoter active in hematopoietic cells may be used. For example, Unwalla et al showed that a nucleic acid may be expressed under the control of one TAR element using an hsp70 promoter.

In the nucleic acid of the invention, the TAR elements can be operably linked to a nucleic acid encoding a transgene. In some aspects of the invention, the TAR elements are linked to a sequence such as a multiple cloning site into which a transgene may be inserted so that the transgene is functionally linked to the TAR elements. Expression of the operably linked nucleic acid is controlled by the TAR elements. Expression includes transcription of the nucleic acid, in particular, complete transcription. In the absence of TAT, the TAR elements block efficient full length transcription of the operably linked nucleic acid. A further step of expression, which is not controlled by the TAR element, may be translation. However, translation may not be required, e.g., if the transgene is an RNA, as described below.

The TAR elements are situated downstream of a promoter, and preferably immediately downstream of the start of transcription. Their arrangement preferably allows expression at the 5' end of a transcript transcribed from the promoter. Karn, 1999 and Muesing et al, 1987, also describe functional positions for a TAR element. As found by Churcher et al, 1995, TAR elements can also be functional if they are located more than 200 nucleotides or several hundred nucleotides downstream of the start of transcription.

Localisation or expression at the 5' end of a transcript transcribed from the promoter in the context of the present invention means that the two or more TAR elements are preferably situated within the first 400 nucleotides of the sequence that is to be transcribed or the transcript, preferably, within the first 300 nucleotides thereof, within the first 250 nucleotides thereof, within the first 200 nucleotides thereof, within the first 150 nucleotides thereof, or within the first 118 nucleotides thereof.

The TAR elements can be directly adjacent or be separated by a spacer. The spacer may comprise 1 - about 350 nucleotides, preferably, 2 - about 300 nucleotides, 3 - about 200 nucleotides, 4 - about 150 nucleotides, about 5 - about 100 nucleotides or about 10 - about 50 nucleotides. The spacer may comprise a recognition site for a restriction enzyme to facilitate cloning. In one embodiment, one of the TAR elements is situated directly at the 5' end of the transcribed sequence. An initiator element (INI) may be situated at the start of the transcribed sequence to allow initiation of transcription (Kam, 1999, Churcher et al, 1995). The initiator element may extend into the non-transcribed sequence upstream of the start of transcription.

In one embodiment, the invention provides a nucleic acid comprising two TAR elements operably linked to a nucleic acid sequence that is capable of providing a therapeutic effect in a cell infected with a retrovirus. The retrovirus is capable of producing TAT. It may be a lentivirus. The lentivirus may be HIV or SIV, in particular, HIV, or HIV-1. A nucleic acid sequence that is capable of providing a therapeutic effect in a cell infected with a retrovirus nucleic acid may be capable of effecting cell-death or capable of effecting excision of at least part of a provirus from the cell, in particular, an HIV provirus. It may alternatively be capable of providing a therapeutic effect through expression in the infected cell. For example, symptoms of the infection may be ameliorated or the immune system activated.

In the context of the invention, the nucleic acid sequence operably linked to the at least two TAR elements may be a nucleic acid encoding a transgene. This transgene may be selected from the group comprising
a) a cytostatic or cytocidal protein such as cytosine deaminase, thymidine kinase, e.g., HSV thymidine kinase, and a toxin such as diphtheria toxin,
b) an antiretroviral protein such as an interferon, e.g., interferon alpha, a ribosome inactivating protein such as trichosanthin, and cyanovirin-N,
c) a tailored recombinase (Tre) capable of recombining asymmetric target sequences within the long terminal repeat (LTR) of proviral DNA, and
d) RNA. An RNA transgene can be, e.g., a Ribozyme, antisense RNA, RNAi or shRNA. shRNA may for example be used to target Rev. The approaches disclosed in US 2006/0122139 can for example be used in the context of two TAR elements.

Nucleic acids encoding transgenes of groups a, b, and d are examples of nucleic acid sequences capable of effecting cell-death. Therapeutic effects, such as cell death of an infected cell, can be effected by the transcript, e.g., in case of an RNA preventing expression of an essential gene, or by a protein encoded by the transcript. An encoded protein may be encoded as a fusion protein. Suitable transgenes can be selected from those mentioned in Ragheb et al., 1999 or the publications cited therein.

Nucleic acids encoding transgenes of group c are nucleic acids capable of effecting excision of at least part of a provirus, e.g., an HIV provirus. The tailored recombinase may be a tailored recombinase described in Sarkar et al., 2007, European patent application 10005499.8 and/or 08706955.5, e.g., Tre 3.0 or Tre 4.0. Tre may recognize the recognition sequence SEQ ID NO:3. Use of a recombinase for eliminating an HIV infection in the context of the invention has the advantage over the methods describe in Ragheb et al., 1999, that the consequence of treatment is not cell death but elimination of the integrated proviral DNA. Thus, cells, or patients, can be cured from HIV infection making use of the present invention.

In situations of gene therapy, it is especially important to control transcription of transgenes, in particular, to prevent or reduce basal transcription in the absence of an inducer of transcription. This contributes to safety of the therapy, which is of course of paramount importance especially where gene therapy of a human is intended. Particulary in cases where the transgene is cytotoxic, the reasons are self evident, however, unwanted expression should also be reduced to avoid, e.g., stimulus for allergic responses.

In particular in the context of gene therapy, specific advantages are provided by a lentiviral vector comprising
a) at least two TAR elements operably linked to a nucleic acid sequence encoding a tailored recombinase (Tre) capable of recombining asymmetric target sequences within the long terminal repeat (LTR) of proviral DNA, and
b) a polyadenylation signal,
wherein the vector does not comprise the recognition sequence of the tailored recombinase. The recognition sequence of the tailored recombinase may be SEQ ID NO:3, e.g., for Tre 3.0. Said sequence is thus in one embodiment not part of the vector of the invention. Instead, the vector sequence may be changed, however, maintaining a sequence comprising essential TAR elements and the polyadenylation signal. In one embodiment, the vector comprises SEQ ID NO:4. Such vectors are resistant to Tre recombinases through complete or partial mutation or deletion of the target sequences from the respective LTR sequence. Preferably, vectors of the invention comprise SIN-LTRs or an HIV-1 derived internal promoter. One, two or more TAR element may comprise SEQ ID NO:1 or another functional TAR sequence. A polyadenylation signal may comprise SEQ ID NO:5.

The nucleic acid of the invention comprising at least two TAR elements may be a retroviral vector, e.g., a SIN vector, comprising a packaging signal. The nucleic acid may be packaged with viral (e.g., HIV) coat proteins. This makes it possible to target the nucleic acid to cells which may be infected with HIV. Such cells may express TAT. It may be advantageous to target the nucleic acid to cells such as resting T cells, wherein HIV is not actively proliferating, in particular, if the encoded transgene is a Tre recombinase.

In a further aspect, the present invention also relates to a pharmaceutical composition comprising a nucleic acid comprising at least two TAR elements operably linked to a nucleic acid such as a transgene. The nucleic acid comprising at least two TAR elements operably linked to a nucleic acid may be the nucleic acid described above. The nucleic acid may be packaged in a virus coat, as known in the art. Preferably, the nucleic acid is formulated to target cells potentially infected by HIV.

The composition may also be suitable or formulated for in vitro administration to cells, in particular, cells isolated from a subject, e.g., a patient infected by HIV. Such cells may be from the hematopoietic lineage, e.g., T-cells such as CD4+ T-cells and/or hematopoietic stem cells such as CD34+ cells. Cells comprising the nucleic acid, in particular, the vector of the invention, may later be administered to a subject, e.g., to the subject the cells were derived from. In one embodiment, the invention is directed to a cell from the hematopoietic lineage isolated from a subject comprising the nucleic acid of the invention, in particular, the lentiviral vector of the invention encoding a TRE recombinase operably linked at least two TAR elements.

The pharmaceutical composition of the invention may comprise a cell comprising a nucleic acid of the invention, in particular for administration to a subject infected with a retrovirus expressing TAT, such as HIV.

The pharmaceutical composition may be for single or multiple administration. The composition may be administered to a subject in need thereof in an effective amount, e.g., an HIV infected human. Effective amounts and a schedule of administration may be determined by the responsible medical practitioner.

In the context of pharmaceutical use, the nucleic acid sequence operably linked to the TAR elements may be a nucleic acid capable of providing a therapeutic effect to cells infected with a retrovirus, or cells comprising TAT. If the nucleic acid sequence operably linked to the TAR elements is a multiple cloning site, the construct can be conveniently used to test the therapeutic effect of agents upon expression in cells comprising TAT, e.g., cells infected with a retrovirus such as HIV.

The invention thus also provides a test kit for the therapeutic effect of RNA or protein agents upon expression in cells comprising TAT, e.g., cells infected with a retrovirus such as HIV, comprising a nucleic acid sequence operably linked to at least two TAR elements, which comprises a multiple cloning site suitable for introduction of a candidate agent, e.g., in the from of SEQ ID NO:6. The nucleic acid preferably is a retroviral vector comprising e.g., a packaging signal. The kit may further comprise buffers and/or packaging cells and/or a vector enabling packaging of the nucleic acid. A candidate nucleic acid of which the therapeutic effect is to be tested can be cloned into the multiple cloning site so that it is operably linked to the at least two TAR elements, and an infected cell may be transfected with the nucleic acid under conditions wherein the candidate nucleic acid is expressed. The therapeutic effect of the candidate nucleic acid or RNA or protein expressed from it can then be assessed.

The pharmaceutical composition may comprise suitable excipients, e.g., water, a buffer, a virus coat protein, a salt, a sugar and/or a preservative. The pharmaceutical composition may be formulated for injection, for inhalation and/or for topical administration. The pharmaceutical composition may comprise additional antiretroviral agents and/or medicaments. The composition may also be formulated for administration with additional antiretroviral agents and/or medicaments. Administration may be simultaneous with, before or after administration of the other agents. The agents may be mixed before administration or administered separately. Examples of other agents are a protease inhibitor, reverse transcriptase inhibitor, entry inhibitor, maturation inhibitor and/or integrase inhibitor. For example, the pharmaceutical composition of the present invention may be for use in combination with HAART (Highly active antiretroviral therapy).

The pharmaceutical composition of the invention is preferably for use in treating a retrovirus infection, in particular, HIV infection. The present invention provides a nucleic acid comprising at least two TAR elements operably linked to a nucleic acid, preferably, a nucleic acid described above, for use in treating a retrovirus infection, in particular, HIV infection. The invention also provides a method of treating a subject in need thereof, e.g., a subject infected with HIV, comprising steps wherein an effective amount of a nucleic acid of the present invention is administered to the subject.

The present invention also provides a recombinant virus construct or a cell comprising a nucleic acid comprising at least two TAR elements operably linked to a nucleic acid, preferably, the nucleic acid described above. The recombinant virus construct comprises the nucleic acid of the invention packaged in virus coat proteins.

In another aspect, the invention provides use of a nucleic acid comprising at least two TAR elements for selectively inducing the expression of a nucleic acid sequence operably linked to the TAR elements, wherein said nucleic acid sequence operably linked to the TAR elements is not expressed in a cell not comprising TAT. The invention also provides use of a nucleic acid comprising at least two TAR elements for improving selective inducability of the expression of a nucleic acid sequence operably linked to the TAR elements compared to a nucleic acid comprising one TAR element, wherein expression of said nucleic acid sequence operably linked to the TAR elements in a cell not comprising TAT is reduced by a factor of at least 2.

The invention also provides use of a nucleic acid comprising at least two TAR elements for inhibiting or for decreasing the expression of a nucleic acid sequence operably linked to the TAR elements in a cell not comprising TAT. Said nucleic acid sequence operably linked to the TAR elements may be selectively expressed in a cell comprising TAT, such as a retrovirus or HIV infected cell. The nucleic acid sequence operably linked to the TAR elements may be capable of effecting a therapeutic effect in a retrovirus infected cell, e.g., by encoding any of the transgenes described above. The nucleic acid used may thus be one of the nucleic acids disclosed above. Alternatively, the nucleic acid may encode for another transgene such as a reporter gene. If the nucleic acid sequence operably linked to the TAR elements is a multiple cloning site, the construct can be conveniently used to test the therapeutic effect of agents upon expression in HIV infected cells.

The invention also provides a method for preventing or reducing expression of a transgene operably linked to a nucleic acid comprising at least two TAR elements, comprising culturing a cell comprising said nucleic acid under conditions wherein no TAT is present. As described above, expression of the transgene in the absence of TAT is significantly reduced by the nucleic acid of the invention comprising at least two TAR elements. Expression can selectively be induced by contacting said cell with TAT.

The present invention also provides a lentiviral vector comprising
a) at least one, preferably two TAR elements, which, preferably, are operably linked to a nucleic acid encoding a Tre recombinase, and
b) a polyadenylation signal,
wherein the vector does not comprise a recognition site for a Tre recombinase. For example, the vector does not comprise SEQ ID NO:3. Instead, the vector may comprise SEQ ID NO:4. This has the advantage that the vector is resistant to the action of the Tre recombinase. The vector may comprise SIN LTRs.

### Figure Legends

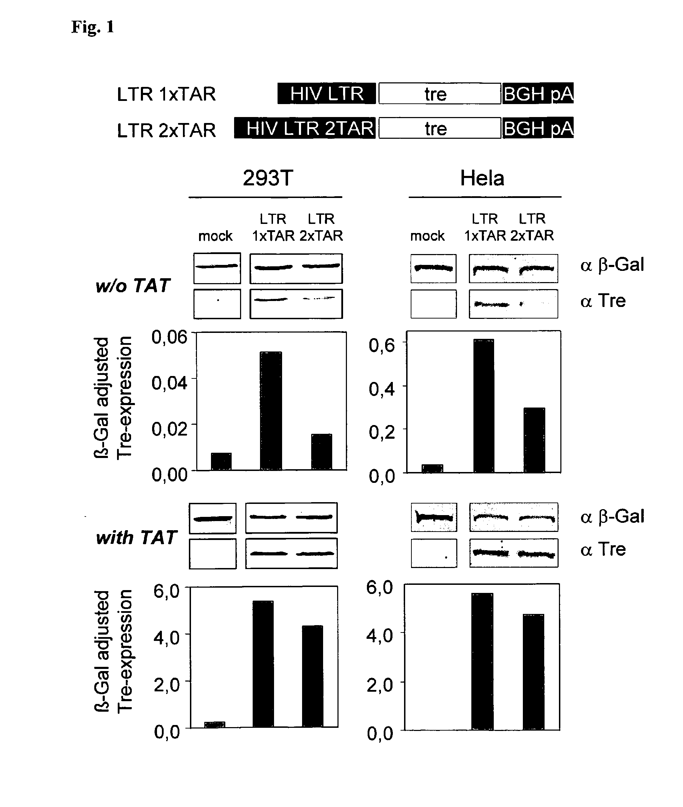

Fig. 1 293T cells and Hela cells were transiently co-transfected with Tre expression plasmid (LTR 1xTAR or LTR 2xTAR), β-Galactosidase expression plasmid and TAT expression plasmid or empty vector (pcDNA3). The backbone of the expression plasmids is derived from vector pcDNA3 (Invitrogen). Shown is the transgene expression cassette containing the Tre recombinase, a polyadenylation site of the bovine growth hormone (BGH polyA) and a HIV-derived LTR promoter containing 1 or 2 TAR elements (LTR 1xTAR/ LTR 2xTAR).

Western Blot analysis was performed to monitor Tre expression in presence and absence of transactivating protein TAT. Analysis of β-Galactosidase expression served as transfection control and was used to normalize Tre signals.

Western Blot analysis and quantification show some basal activity of the LTR promoter in absence of TAT. However, basal activity of the promoter is distinctly decreased when a second TAR element is present in the LTR promoter. In the presence of TAT, comparable amounts of Tre recombinase are expressed in 293T cells and Hela cells transfected with LTR 1xTAT or LTR 2xTAT.

Fis. 2
A) The sequence of TAT-inducible promoter LTR 2xTAR is shown (SEQ ID NO:6), wherein bold letters indicate that the residue belongs to the LTR promoter, underlined letters indicate that the residue belongs to a TAR element, and italic indicates the multiple cloning site.
B) The sequence of a transgene encoding a Tre recombinase was cloned into the multiple cloning site of SEQ ID NO:6, leading to SEQ ID NO: 7 shown in Fig. 4B A, wherein bold letters indicate that the residue belongs to the LTR promoter, underlined letters indicate that the residue belongs to a TAR element, and italic indicates the Tre recombinase open reading frame.

### Examples

### 1. Generation of a double TAR HIV-Promoter

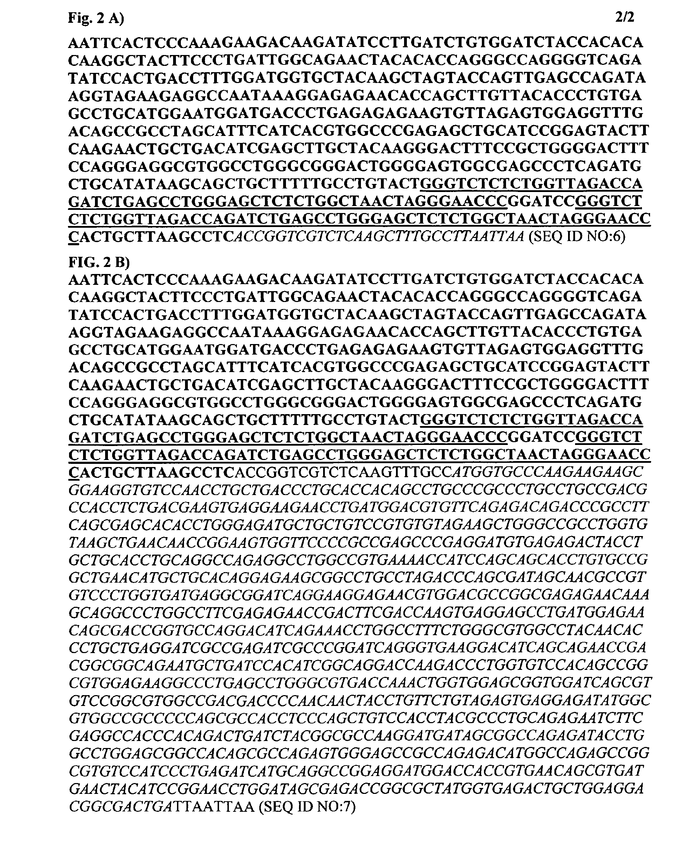

Clinical isolate HIV-1 NL4-3 was used as a template for PCR, and two HIV-LTR fragments were generated (fragment 1: -443 to +59/ fragment 2: +1 to +73) (sequence refers to the 3'LTR of HIV-1 clinical isolate NL-4-3 NCBI accession number AF324493). At the 3' end of fragment 1 and at the 5' end of fragment 2, a *BamH*I site was introduced, which was used for further cloning. The two fragments were digested with *BamH*I and ligated, resulting in TAT-inducible promoter LTR 2xTAR. The resulting sequence is shown in Fig. 2A (SEQ ID NO:6).

A nucleic acid that is capable of providing a therapeutic effect in a cell infected with a retrovirus, in particular, a transgene encoding a Tre recombinase was cloned into the multiple cloning site, leading to SEQ ID NO: 7 shown in Fig. 2B.

### 2. Generation of lentiviral SIN L TR with mutated Tre3.0 recognition site

The recognition site of Tre 3-0, which is conserved in a wide majority of all HIV-1 B-type LTRs, was mutated from 5'-AACCCACTGCTTAAGCCTCAATAAAGCTTGCCTT-3' (SEQ ID NO:3) to 5'-AACCCTGACGAATTCGGAGAATAAACGAACGGAA-3' (SEQ ID NO:4), leaving the nucleotides belonging to the Poly-A Signal (5'-AATAAA-3', SEQ ID NO:5) and residues of the TAR element (5'-AACCC-3', SEQ ID NO:2) unchanged.

Sequence mutations were introduced by fusionPCR, and cloned into lentiviral LTRs using standard cloning techniques.

### 3. Analysis of transgene expression from vectors comprising one and two TAR elements in presence and absence of TAT

To analyze the promoter activity of the different LTR promoters under overexpressing conditions, 293T cells and Hela cells were transiently co-transfected with Tre expression plasmid (LTR 1xTAR or LTR 2xTAR), β-Galactosidase expression plasmid and TAT expression plasmid or empty vector. 48 h after transfection, cells were harvested and 12.5 - 25 µg of total protein were separated by SDS PAGE. Western Blot analysis was performed to monitor Tre expression in the presence and absence of transactivating protein TAT. Analysis of β-Galactosidase expression served as transfection control and was used to normalize Tre signals. Western Blot signals were recorded and quantified using the Bioscience LI-COR Odyssey Infrared Imaging System.

Western Blot analysis and quantification show that even in the absence of TAT, there is some basal activity of the LTR promoter resulting in low levels of Tre expression in 293T cells or Hela cells transfected with Tre expression plasmids. However, basal activity of the promoter can be distinctly decreased by introduction of a second TAR element to the LTR promoter. On the other hand, in the presence of TAT, comparable amounts of Tre recombinase are expressed in 293T cells and Hela cells transfected with LTR 1xTAT or LTR 2xTAT.

### Literature

Berkhout et al., 1990, Nucleic Acids Research 18, 1839-1846.
Braddock et al., 1994, J. Virol 68, 8396-8400.
Caruso et al., 1997, Virus Res. 52, 133-143.
Churcher et al., 1995, Proc. Natl. Acad. Sci. USA 92, 2408-2412.
Emiliani et al, 1994, AIDS Res Hum Retrovir10, 1751-1752.
Emiliani et al, 1996, J. Biomed. Sci 3, 31-40.
Fenrick et al., 1989, J. Virol. 63, 5006-5012.
Karn, J., 1999. J. Mol. Biol. 293, 235-254.
Kao et al., 1987, Nature 330, 489-493.
Michienzi, et al., 2002. Proc. Natl. Acad. Sci. U. S. A 99, 14047-14052.
Muesing, M. A., et al., 1987. Cell 48, 691-701.
Park et al., 2009, Virus Res. 140, 112-120.
Ragheb, J.A., et al., 1999. Hum. Gene Ther. 10, 103-112.
Roy, S., et al., 1990. Genes Dev. 4, 1365-1373
Dull, T., et al., 1998. J. Virol. 72, 8463-8471.
Sarkar, I., et al F., 2007. Science 316, 1912-1915.
Schambach, A., et al., 2007. Mol. Ther. 15, 1167-1173.
Selby et al., 1989, Genes Dev. 3, 547-558.
Sodorski et al., 1985, Science 227, 171-173.
Unwalla et al., 2004, Nat. Biotech 22, 1573-1578.
Unwalla et al., 2008, Current HIV/AIDS Reports 5, 40-43.
US 20060122139
EP 10005499.8
EP 08706955.5

## Claims

1. A nucleic acid comprising at least two TAR elements operably linked to a nucleic acid sequence that is capable of providing a therapeutic effect in a cell infected with a retrovirus selected from a group comprising HIV.

2. The nucleic acid of claim 1, wherein the at least two TAR elements are suitable for inhibiting the expression of the nucleic acid sequence operably linked to the TAR elements in a cell not comprising TAT.

3. The nucleic acid of any of claims 1 or 2, wherein the at least two TAR elements are suitable for selectively inducing the expression of the nucleic acid sequence operably linked to the TAR elements, wherein expression of said nucleic acid sequence operably linked to the TAR elements is significantly decreased in a cell not comprising TAT compared to a nucleic acid comprising one TAR element operably linked to the nucleic acid sequence.

4. The nucleic acid of any of the preceding claims, wherein said nucleic acid sequence operably linked to the TAR elements may be selectively expressed in a cell comprising TAT, such as an HIV-infected cell.

5. The nucleic acid of any of the preceding claims, wherein the nucleic acid comprising at least two TAR elements is an RNA or a DNA, in particular, a vector such as a retroviral vector or a lentiviral vector.

6. The nucleic acid of any of the preceding claims, wherein said nucleic acid sequence operably linked to the TAR elements is a nucleic acid encoding a transgene selected from the group comprising
a) a cytostatic or cytocidal protein such as cytosine deaminase, thymidine kinase, e.g., HSV thymidine kinase, or a toxin such as diphtheria toxin,
b) an antiretroviral protein such as an interferon, e.g., interferon alpha,
c) a tailored recombinase (Tre) capable of recombining asymmetric target sequences within the long terminal repeat (LTR) of proviral DNA, and
d) RNA.

7. The nucleic acid of any of the preceding claims, wherein the nucleic acid is a lentiviral vector comprising at least two TAR elements operably linked to a nucleic acid sequence encoding a tailored recombinase (Tre) capable of recombining asymmetric target sequences within the long terminal repeat (LTR) of proviral DNA, wherein the vector comprises
a) at least two TAR elements, and
b) a polyadenylation signal,
wherein the vector does not comprise the recognition sequence of the tailored recombinase, wherein the recognition sequence optionally is SEQ ID NO:3,
wherein the vector preferably comprises SEQ ID NO:4.

8. A pharmaceutical composition comprising a nucleic acid comprising at least two TAR elements operably linked to a nucleic acid, which preferably is the nucleic acid of any of claims 1 to 7.

9. The pharmaceutical composition of claim 8, wherein the composition is for use in treating a retrovirus infection, in particular, HIV infection.

10. The pharmaceutical composition of any of claims 8 or 9, wherein the composition comprises additional antiretroviral agents, or wherein the composition is for administration in combination with additional antiretroviral agents.

11. A nucleic acid comprising at least two TAR elements operably linked to a nucleic acid, preferably, the nucleic acid of any of claims 1 to 8, for use in treating a retrovirus infection, in particular, HIV infection.

12. A recombinant virus construct or a cell comprising a nucleic acid of any of claims 1 to 8.

13. Use of a nucleic acid comprising at least two TAR elements for inhibiting or decreasing the expression of a nucleic acid sequence operably linked to the TAR elements in a cell not comprising TAT.

14. Use of a nucleic acid comprising at least two TAR elements for improving selective inducability of the expression of a nucleic acid sequence operably linked to the TAR elements compared to a nucleic acid comprising one TAR element, wherein expression of said nucleic acid sequence operably linked to the TAR elements in a cell not comprising TAT is reduced by a factor of at least 2.

15. The use of any of claims 13 or 14, wherein said nucleic acid sequence operably linked to the TAR elements may be selectively expressed in a cell comprising TAT, such as an HIV-infected cell.

16. The use of any of claims 13 to 15, wherein the nucleic acid is the nucleic acid of any of claims 1 to 7.

17. A lentiviral vector comprising
a) at least one, preferably two TAR elements, and
b) a polyadenylation signal,
wherein the vector does not comprise SEQ ID NO:3,
wherein the vector preferably comprises SEQ ID NO:4.


SEQUENCE LISTING

<110>  Heinrich-Pette-Institut Leibniz-Zentrum für experimentelle
       Immunologie

<120>  Lentiviral vector comprising at least two TAR elements

<130>  P83359 SM

<160>  7

<170>  PatentIn version 3.5

<210>  1
<211>  59
<212>  RNA
<213>  Human immunodeficiency virus type 1

<400>  1
gggucucucu gguuagacca gaucugagcc ugggagcucu cuggcuaacu agggaaccc        59

<210>  2
<211>  5
<212>  DNA
<213>  Human immunodeficiency virus

<400>  2
aaccc                                                                    5

<210>  3
<211>  34
<212>  DNA
<213>  Human immunodeficiency virus

<400>  3
aacccactgc ttaagcctca ataaagcttg cctt                                   34

<210>  4
<211>  34
<212>  DNA
<213>  Artificial Sequence

<220>
<223>  TAR sequence without TRE recognition site

<400>  4
aaccctgacg aattcggaga ataaacgaac ggaa                                   34

<210>  5
<211>  6
<212>  DNA
<213>  Artificial Sequence

<220>
<223>  polyadenylation site

<400>  5
aataaa                                                                   6

<210> 6
<211> 611
<212> DNA
<213> Artificial Sequence

<220>
<223> sequence including 2 TAR elements

<400> 6
aattcactcc caaagaagac aagatatcct tgatctgtgg atctaccaca cacaaggcta 60
cttccctgat tggcagaact acacaccagg gccaggggtc agatatccac tgacctttgg 120
atggtgctac aagctagtac cagttgagcc agataaggta gaagaggcca ataaaggaga 180
gaacaccagc ttgttacacc ctgtgagcct gcatggaatg gatgaccctg agagagaagt 240
gttagagtgg aggtttgaca gccgcctagc atttcatcac gtggcccgag agctgcatcc 300
ggagtacttc aagaactgct gacatcgagc ttgctacaag ggactttccg ctggggactt 360
tccagggagg cgtggcctgg gcgggactgg ggagtggcga gccctcagat gctgcatata 420
agcagctgct ttttgcctgt actgggtctc tctggttaga ccagatctga gcctgggagc 480
tctctggcta actagggaac ccggatccgg gtctctctgg ttagaccaga tctgagcctg 540
ggagctctct ggctaactag ggaacccact gcttaagcct caccggtcgt ctcaagcttt 600
gccttaatta a 611

<210> 7
<211> 1666
<212> DNA
<213> Artificial Sequence

<220>
<223> sequence comprising 2 TAR elements and transgene

<400> 7
aattcactcc caaagaagac aagatatcct tgatctgtgg atctaccaca cacaaggcta 60
cttccctgat tggcagaact acacaccagg gccaggggtc agatatccac tgacctttgg 120
atggtgctac aagctagtac cagttgagcc agataaggta gaagaggcca ataaaggaga 180
gaacaccagc ttgttacacc ctgtgagcct gcatggaatg gatgaccctg agagagaagt 240
gttagagtgg aggtttgaca gccgcctagc atttcatcac gtggcccgag agctgcatcc 300
ggagtacttc aagaactgct gacatcgagc ttgctacaag ggactttccg ctggggactt 360
tccagggagg cgtggcctgg gcgggactgg ggagtggcga gccctcagat gctgcatata 420
agcagctgct ttttgcctgt actgggtctc tctggttaga ccagatctga gcctgggagc 480
tctctggcta actagggaac ccggatccgg gtctctctgg ttagaccaga tctgagcctg 540
ggagctctct ggctaactag ggaacccact gcttaagcct caccggtcgt ctcaagtttg 600
ccatggtgcc caagaagaag cggaaggtgt ccaacctgct gaccctgcac cacagcctgc 660
ccgccctgcc tgccgacgcc acctctgacg aagtgaggaa gaacctgatg gacgtgttca 720
gagacagacc cgccttcagc gagcacacct gggagatgct gctgtccgtg tgtagaagct 780
gggccgcctg gtgtaagctg aacaaccgga agtggttccc cgccgagccc gaggatgtga 840
gagactacct gctgcacctg caggccagag gcctggccgt gaaaaccatc cagcagcacc 900
tgtgccggct gaacatgctg cacaggagaa gcggcctgcc tagacccagc gatagcaacg 960
ccgtgtccct ggtgatgagg cggatcagga aggagaacgt ggacgccggc gagagaacaa 1020
agcaggccct ggccttcgag agaaccgact tcgaccaagt gaggagcctg atggagaaca 1080
gcgaccggtg ccaggacatc agaaacctgg cctttctggg cgtggcctac aacaccctgc 1140
tgaggatcgc cgagatcgcc cggatcaggg tgaaggacat cagcagaacc gacggcggca 1200
gaatgctgat ccacatcggc aggaccaaga ccctggtgtc cacagccggc gtggagaagg 1260
ccctgagcct gggcgtgacc aaactggtgg agcggtggat cagcgtgtcc ggcgtggccg 1320
acgaccccaa caactacctg ttctgtagag tgaggagata tggcgtggcc gcccccagcg 1380
ccacctccca gctgtccacc tacgccctgc agagaatctt cgaggccacc cacagactga 1440
tctacggcgc caaggatgat agcggccaga gatacctggc ctggagcggc cacagcgcca 1500
gagtgggagc cgccagagac atggccagag ccggcgtgtc catccctgag atcatgcagg 1560
ccggaggatg gaccaccgtg aacagcgtga tgaactacat ccggaacctg gatagcgaga 1620
ccggcgctat ggtgagactg ctggaggacg gcgactgatt aattaa 1666